(19) Europäisches Patentamt
European Patent Office
Office européen des brevets

(11) EP 2 226 070 B1

(12) **FASCICULE DE BREVET EUROPEEN**

(45) Date de publication et mention
de la délivrance du brevet:
**06.05.2015 Bulletin 2015/19**

(51) Int Cl.:
*A61K 31/00* (2006.01)    *A61P 7/02* (2006.01)
*G06F 19/00* (2011.01)

(21) Numéro de dépôt: **10002066.8**

(22) Date de dépôt: **01.03.2010**

(54) **Procédé permettant de déterminer le dosage optimal d'antivitamines K d'un traitement anticoagulant**

Prozess für Bestimmung der optimalen Dosierung von Antivitamin K in antithrombotischer Behandlung

Process for determining the optimal dosage of antivitamins K in anticoagulant therapy

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **02.03.2009 FR 0951306**

(43) Date de publication de la demande:
**08.09.2010 Bulletin 2010/36**

(73) Titulaire: **Puech, Roger**
**81200 Mazamet (FR)**

(72) Inventeur: **Puech, Roger**
**81200 Mazamet (FR)**

(74) Mandataire: **Richebourg, Michel François**
**Cabinet Michel Richebourg**
**"Le Clos du Golf"**
**69, rue Saint-Simon**
**42000 Saint Etienne (FR)**

(56) Documents cités:
• **HÔPITAL D'ALBI: "Recommandations de prescription des AVK"**, [Online] janvier 2004 (2004-01), XP002543142 Extrait de l'Internet: URL:http://www.geocities.com/bpradines/guideAVK.doc> [extrait le 2009-08-26]
• **SCOTTISH INTERCOLLEGIATE GUIDELINES NETWORK (SIGN): "Antithrombotic therapy: Quick reference guide"**, [Online] mars 1999 (1999-03), XP002543143 Extrait de l'Internet: URL:http://www.sign.ac.uk/pdf/qrg36.pdf> [extrait le 2009-08-26]
• **BRADLOW B A: "Warfarin therapy - a practical guide" SOUTH AFRICAN MEDICAL JOURNAL, vol. 64, no. 9, 27 août 1983 (1983-08-27), pages 317-319, XP009121843**
• **WHITE R H; HONG R; VENOOK A P; DASCHBACH M M; MURRAY W; MUNGALL D R; COLEMAN R W: "INITIATION OF WARFARIN THERAPY COMPARISON OF PHYSICIAN DOSING WITH COMPUTER-ASSISTED DOSING" JOURNAL OF GENERAL INTERNAL MEDICINE, vol. 2, no. 3, 1987, pages 141-148, XP009121821**
• **VERGNES C: "Surveillance des traitements antithrombotiques" REVUE FRANÇAISE DES LABORATOIRES, EDITIONS SCIENTIFIQUES ET MEDICALES ELSEVIER, vol. 1995, no. 272, 1 janvier 1995 (1995-01-01), pages 89-99, XP004895448 ISSN: 0338-9898**
• **HASSO SCHOLZ & ULRICH SCHWABE: "Taschenbuch der Arzneibehandlung (Angewandte Pharmakologie)" 2005, SPRINGER, XP002543144 * page 775, colonne de droite - page 776, colonne de gauche ***
• **CARTER B L; TAYLOR J W; BECKER A: "EVALUATION OF THREE DOSAGE-PREDICTION METHODS FOR INITIAL IN-HOSPITAL STABILIZATION OF WARFARIN THERAPY" CLINICAL PHARMACY, vol. 6, no. 1, 1987, pages 37-45, XP009121823**

Remarques:
Le dossier contient des informations techniques présentées postérieurement au dépôt de la demande et ne figurant pas dans le présent fascicule.

**Description**

DOMAINE D'APPLICATION DE L'INVENTION

La présente invention a trait au domaine médical et concerne plus particulièrement les méthodes de contrôle et de surveillance biologiques d'un traitement par anticoagulants.

DESCRIPTION DE L'ART ANTERIEUR

La coagulation sanguine naturelle est régulée par la présence de divers facteurs de coagulation qui de concert se chargent d'un équilibre normal entre les tendances naturelles à la coagulation et celles à l'anticoagulation. La coagulation est cependant un phénomène physiologique complexe qui peut aussi aboutir à la formation d'un caillot.
Dans certains cas pathologiques, le processus de coagulation doit donc être corrigé ou modifié à l'aide d'un anticoagulant destiné à empêcher ou à retarder la coagulation du sang, en traitement ou en prévention de certains troubles et le degré d'anticoagulation lors de l'utilisation de ces médicaments dépend aussi d'un grand nombre de facteurs comme d'autres médicaments pris en même temps et l'alimentation du patient traité.
Les anticoagulants sont des médicaments utilisés dans le traitement et la prévention des thromboses (caillots sanguins dans la circulation générale) et des embolies (caillot sanguin dans la circulation artérielle pulmonaire). Les plus connus sont l'héparine et ses dérivés et les antivitamines K plus communément connues sous l'abréviation "AVK". Les premières agissent directement sur la coagulation, rapidement et sont injectées par voie sous-cutanée ou à l'intérieur d'une veine (intraveineuse). Les antivitamines K ont pour but d'empêcher la synthèse, par la glande hépatique (le foie) de facteurs ayant une activité sur la coagulation. Les antivitamines K ont un délai d'action, par rapport aux héparines, beaucoup plus long.
Un antivitamine K est un médicament anticoagulant qui ralentit la coagulation sanguine qui agit en s'opposant à l'action de la vitamine K, vitamine indispensable à la coagulation sanguine. Il s'administre par voie orale (comprimés), généralement pour une durée prolongée (plusieurs semaines, mois, voire toute la vie pour certaines indications). Son effet s'installe progressivement en 2 à 4 jours et disparaît également progressivement en quelques jours après l'arrêt du traitement.
La prise d'antivitamines K expose le patient à deux risques principaux :

- l'hémorragie liée à un surdosage,
- la thrombose liée à un sous-dosage.

En début de traitement, il faut identifier la dose appropriée qui convient à chaque patient. Sa complication principale est la survenue d'hémorragies en cas de surdosage : la dose doit donc être suffisante pour éviter le risque de thrombose et ne doit pas être excessive pour éviter le risque hémorragique.
De plus, la même dose d'antivitamines K ne provoque pas le même ralentissement de la coagulation chez tous les patients. Ceci implique une adaptation du dosage à la situation et un contrôle régulier du degré d'anticoagulation au moyen d'un examen sanguin.
La surveillance régulière tout au long du traitement pour éviter un surdosage avec risque d'hémorragie, ou un sous-dosage avec risque de thrombose, se fait par la mesure au niveau du sang d'un paramètre appelé INR. L'INR ("International Normalized Ratio" ou Rapport Normalisé International) est un test de laboratoire réalisé à partir d'un prélèvement de sang pour assurer la mesure du temps de Quick. Il mesure ainsi le temps de coagulation d'un patient et le compare à celui d'un sujet qui ne reçoit pas de traitement à base d'un antivitamine K. Chez un sujet non traité, l'INR est considéré égal à 1. Chez un patient traité par un antivitamine K, l'INR augmente en fonction de l'anticoagulation. Il sera d'autant plus élevé que le sang est anticoagulé. L'INR permet ainsi d'évaluer l'efficacité du traitement par un antivitamine K.
Dans la plupart des études de cas, il a été déterminé que l'INR dit cible doit se situer entre 2 et 3 (ce qui correspond à un sang qui mettra 2 à 3 fois plus de temps à coaguler que celui d'un sujet non traité par antivitamine K). Lorsque l'INR est inférieur à 2, la dose est insuffisante. Lorsque l'INR est supérieur à 3, l'anticoagulation devient excessive. Dans certaines circonstances, il peut être souhaitable d'obtenir un INR plus élevé compris entre 3 et 4,5. Dans tous les cas, un INR supérieur à 4 est associé à un risque accru d'hémorragie. En fonction de la pathologie du patient, c'est toujours le médecin qui prescrit et adapte l'INR souhaité au cours du traitement.
En début de traitement, l'INR doit être mesuré fréquemment afin d'identifier la dose de l'antivitamine K prescrit qui convient jusqu'à ce que l'INR «cible» optimal soit obtenu et maintenu constant, et ce après plusieurs tentatives de dosage. Une fois la dose appropriée déterminée, la fréquence du contrôle de l'INR peut diminuer progressivement, puisque l'INR cible reste souvent constant. Il devra être effectué au minimum une fois par mois. Toutefois, certaines circonstances particulières peuvent provoquer un déséquilibre du traitement, en augmentant ou au contraire en diminuant son effet anticoagulant. Ces circonstances, principalement représentées par la prise simultanée e certains médicaments ou par une alimentation riche en vitamines K, nécessiteront alors des contrôles supplémentaires de l'INR afin d'adapter le dosage.

Actuellement, la méthode traditionnelle pour obtenir et maintenir constant l'INR cible qui se situe entre 2 et 3 (mais qui peut être aménagé en fonction de la maladie pour laquelle le traitement est prescrit), est la suivante.

Le médecin diagnostique une dose initiale d'un antivitamine K, toujours probatoire, qui doit être aussi proche que possible de la dose d'équilibre préconisée par les laboratoires fournissant ledit antivitamine K. Elle est habituellement de 20 mg (1 comprimé exprimé en 4/4 de dose) par jour soit 28/4, à adapter en fonction des résultats biologiques connus. Chez les patients à risque hémorragique particulier, la dose initiale est prescrite habituellement plus faible.

La dose d'équilibre pour atteindre l'INR cible sera déterminée en adaptant la dose initiale en fonction de l'INR mesuré avec un ajustement de posologie s'effectuant par paliers de 5 mg (soit 1/4 de comprimé).

Le premier contrôle doit s'effectuer dans les 48 +/-12 heures après la première prise d'antivitamines K, pour dépister une hypersensibilité individuelle : un INR supérieur à 2 annonce un surdosage à l'équilibre et doit faire réduire la posologie.

Le deuxième contrôle s'effectue en fonction des résultats du premier INR, pour apprécier l'efficacité anticoagulante (selon les cas entre 3 à 6 jours après la première prise).

Les contrôles ultérieurs doivent être pratiqués tous les 2 à 4 jours jusqu'à stabilisation de l'INR, puis avec un espacement progressif jusqu'à un intervalle maximal de 1 mois.

Selon cette méthode dite empirique, l'équilibre du traitement par antivitamines K pour atteindre et maintenir l'INR cible peut demander plusieurs semaines, voire plusieurs mois avec les risques encourus de thrombose ou de complication hémorragique, en cas d'un sous-dosage ou d'un surdosage non rectifié à temps.

L'inconvénient de cette méthode est qu'elle réclame pour chaque mesure d'INR, le cycle de contrôle suivant qui va du thérapeute qui prescrit (souvent le médecin généraliste), à celui qui prélève un échantillon sanguin (souvent l'infirmier), puis au thérapeute qui analyse en laboratoire l'échantillon prélevé pour mesurer L'INR (souvent le pharmacien biologiste), et enfin à nouveau au thérapeute prescripteur qui interprète les résultais obtenus pour adapter la posologie afin d'engager un nouveau cycle de contrôle.

On comprend que ces cycles de contrôle successivement répétés pour atteindre par tâtonnements la posologie adéquate exigent le suivi, la réactivité et la coordination très importants entre tous les thérapeutes intervenants et le patient traité ce qui pose des problèmes car ces thérapeutes exercent le plus souvent indépendamment les une des autres tant sur le plan déontologique que sur le plan géographique. Outre l'incertitude et les délais importants pour déceler le dosage optimal pour atteindre et maintenir l'INR cible, la méthode traditionnelle ne satisfait jamais les thérapeutes qui préfèrent se rejeter les responsabilités (mauvaise prescription, prélèvement dans de mauvaises conditions, incertitude des mesures, etc...) des risques encourus plutôt que de collaborer pour trouver des solutions plus radicales.

Plusieurs publications ont déjà traité du dosage des antivitamines K.

- HÔPITAL D'ALBI: "Recommandations de prescription des AVK" [Online] janvier 2004 (2004-01), XP002543142 Extrait de l'Internet: URL:http://www.geocities.com/bpradines/gui deAvk.doc [extrait le 2009-08-26J. Cette publication divulgue la posologie initiale recommandée pour des différents antivitamines K ainsi qu'un ajustement du traitement basé sur les valeurs de l'INR mesurés en fonction du temps (page 6, "Quelle molécule choisir" et "Ajustement du traitement AVK).
- SCOTTISH INTERCOLLEGIATE GUIDELINES NETWORK (SIGN): "Antithrombotic therapy: Ouick reference guide"[Online] mars 1999 (1999-03), XP002543143 Extrait de l'Internet: URL:http://www.sign.ac.uk/pdf/qrg36.pdf [extrait le 2009-08-26]. Cette publication décrit les doses recommandées pour les médicaments antithrombotiques (page 4, colonne de gauche).
- BRADLOW BA: "Warfarin therapy - a practical guide" SOUTH AFRICAN MEDICAL JOURNAL, vol. 64, no. 9, 27 août 1983 (1983-08-27), pages 317-319, XP009121843. Cette publication spécifie la dose initiale recommandée pour la warfarine est de 10 mg / 3 jours et la dose journalière moyenne de 4 - 6,5 mg avec détermination des doses suivantes en utilisant des tests en laboratoire ou biologiques (page 317, colonne de gauche, "Dosage of warfarin").
- WHITE R H; HONG R; VENOOK A P; DASCHBACH M M; MURRAY W; MUNGALL D R; COLEMAN R W: "INITIATION OF WARFARIN THERAPY COMPARISON OF PHYSICIAN DOSING WITH COMPUTER-ASSISTED DOSING" JOURNAL OF GENERAL INTERNAL MEDICINE, vol. 2, no. 3, 1987, pages 141-148, XP009121821. Cette publication décrit un logiciel informatique utilisé par des ordinateurs qui permet de déterminer le dosage de la warfarine en fonction du taux de prothrombine (page 142, colonne de gauche, "Computer program").
- VERGNES C: "Surveillance des traitements antithrombotiques" REVUE FRANÇAISE DES LABORATOIRES, EDITIONS SCIENTIFIOUES ET MEDICALES ELSEVIER, vol. 1995, no. 272, 1 janvier 1995 (1995-01-01), pages 89-99, XP0048954481SSN: 0338-9898. Cette publication se rapporte aux dosages recommandés pour les antivitamines K (Table IV; page 95, colonne de gauche, point 5).
- HASSO SCHOLZ & ULRICH SCHWABE: "Taschenbuch der Arzneibehandlung (Angewandte Phannakologie)" 2005, SPRINGER, XP002543144. Cette publication décrit la dose recommandée pour la phenprocoumone (page 775, colonne de droite - page 776, colonne de gauche).

- CARTER B L; TAYLOR J W; BECKER A: "EVALUATION OF THREE DOSAGE-PREDICTION METHODS FOR INITIAL IN-HOSPITAL STABILIZATION OF WARFARIN THERAPY" CLiNICAL PHARMACY, vol. 6, no. 1, 1987, pages 37-45, XP00912182. Cette publication traite d'une évaluation sur des patients de trois méthodes différentes de prévisions de dosage de la warfarine.

En fait, toutes ces publications divulguent également d'une manière générale, les doses initiales recommandées des différents AVK afin d'obtenir l'INR cible mais impliquent toujours de mettre en oeuvre la méthode traditionnelle dite empirique pour obtenir le dosage optimal adapté à chaque patient et à chaque pathologie.

En conclusion, pour une efficacité optimale tout en minimisant le risque d'hémorragie ou de thrombose, il est essentiel de déterminer rapidement le dosage optimal pour atteindre l'INR cible et de surveiller son maintien par un contrôle régulier de l'INR, et ce, si possible, en effectuant ces opérations dans le même laboratoire.

## DESCRIPTION DE L'INVENTION

Partant de ces constats, et d'un cahier des charges préétabli, le demandeur a mené des recherches qui ont abouti à une composition d'antivitamine K pour l'utilisation dans le traitement anticoagulant qui est établie à partir d'un procédé de détermination de dosage optimal d'un antivitamine K prescrit pour un traitement anticoagulant permettant d'atteindre et de maintenir constant beaucoup plus rapidement que jusqu'à aujourd'hui, un INR cible fixé préalablement par le médecin prescripteur.

Ce procédé de détermination est original en ce qu'il comprend les étapes suivantes consistant :

- (a) à enregistrer la valeur $I_O$ de l'INR optimal cible,
- (b) à enregistrer les valeurs d'INR mesurées dans une période donnée $T_1$ à partir d'un surdosage Dmaxi dudit antivitamine K fixé préalablement par le prescripteur au-dessus du dosage préconisé, afin de déterminer une première pente $P_1$ des valeurs d'INR en fonction du temps $T_1$,
- (c) à partir de la pente $P_1$, à extrapoler et à enregistrer une valeur $I_{maxi}$ d'INR théoriquement atteinte à partir du jour où la valeur $I_O$ de l'INR cible est atteinte et au bout d'une période déterminée $T_0$,
- (d) à enregistrer les valeurs d'INR mesurées dans une période donnée $T_2$ à partir d'un sous-dosage Dmini dudit antivitamine K fixé par le prescripteur au-dessous du dosage préconisé, afin de déterminer une deuxième pente $P_2$ des valeurs d'INR en fonction du temps $T_2$,
- (e) à partir de la pente P2, à extrapoler et à enregistrer une valeur $I_{mini}$ d'INR théoriquement atteinte à partir du jour où la valeur $I_O$ de l'INR cible est atteinte et au bout de la même période déterminée $T_0$,
- (f) à calculer à partir de la différence entre les valeurs $I_{maxi}$ et $I_{mini}$ d'INR théoriquement calculées, un facteur de correction de posologie dudit antivitamine K exprimé selon la formule suivante :

$$F = \frac{I_{maxi} - I_{mini}}{D_{maxi} - D_{mini}}$$

- (g) afin de déterminer à partir de la différence entre les valeurs $I_0$ de l'INR cible optimal et de l'$I_{mini}$ de l'INR minimum calculé, et dudit facteur F de correction de posologie dudit antivitamine K, la posologie adéquate $D_0$ permettant d'atteindre et de maintenir constante la valeur Io de l'INR cible fixée préalablement selon la relation suivante :

$$D_0 = D_{mini} + \frac{I_0 - I_{mini}}{F}.$$

Ce nouveau concept de détermination rapide et calculée du dosage optimal $D_0$ d'un antivitamine K a été décelé par le demandeur, biologiste de formation, qui est parti du constat que les médecins qui prescrivent les dosages d'antivitamines K ne tiennent compte que de la valeur absolue de l'INR alors que ces valeurs décrivent pour un même antivitamine K une trajectoire linéaire qu'il est possible d'extrapoler à partir de très peu de données.

En effet, le demandeur a pu constater que :

- pour un même surdosage d'un antivitamine K, la courbe d'INR est linéaire selon une pente ascendante,
- pour un même sous-dosage d'un antivitamine K, la courbe d'INR est linéaire selon une pente descendante,
- pour un même dosage optimal d'un antivitamine K, la courbe d'INR est linéaire selon une droite horizontale.

Ainsi, à partir d'un INR cible $I_o$ et de deux dosages $D_{maxi}$ et $D_{mini}$ d'un antivitamine K fixés par le prescripteur selon l'état de santé du patient au-dessus et au-dessous du dosage préconisé dudit antivitamine K prescrit, ce procédé a pour avantage de permettre en laboratoire de faire directement une détermination rapide et calculée du dosage optimal $D_0$ dudit antivitamine K, en effectuant seulement deux mesures d'INR à partir de deux prélèvements sanguins peu espacés, le premier après une première période $T_1$ avec administration du surdosage $D_{maxi}$ dudit antivitamine K pour déterminer la pente ascendante $P_1$ et le second après une période donnée $T_2$ avec administration d'un sous-dosage $D_{mini}$ dudit antivitamine K pour déterminer la pente descendante P2.

C'est aussi à partir de son procédé de détermination de dosage optimal d'un antivitamine K, que le demandeur a pu établir également une corrélation proportionnelle entre différents antivitamines K (1/4 de comprimé de "Préviscan" correspond à 0,82 mg de "Sintrom", à 2 mg de "Coumadine" ou à 0,82 mg de "Apegmone") de sorte qu'à partir d'un dosage optimal d'un antivitamine K prescrit, il peut calculer le dosage optimal d'un autre antivitamine K en remplacement du premier.

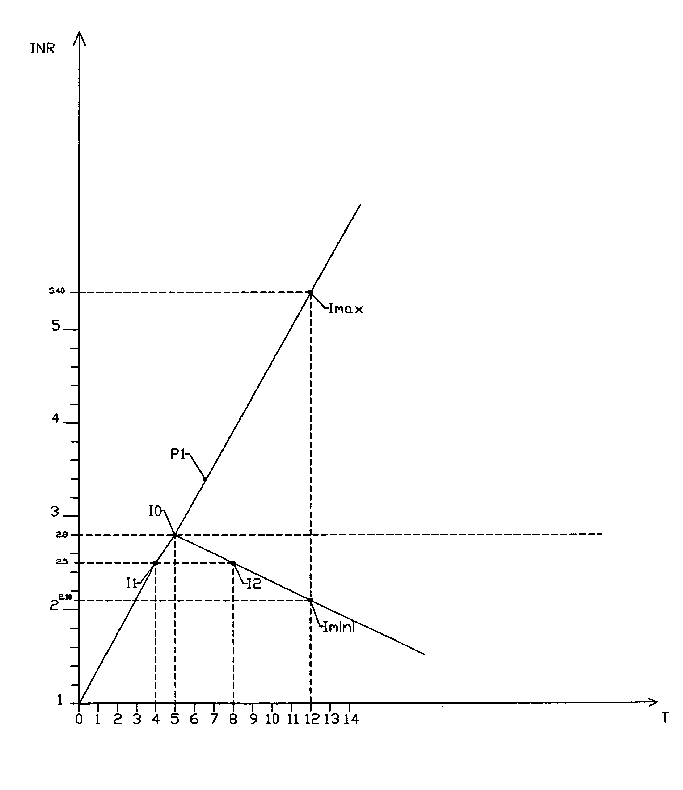

Les concepts fondamentaux de l'invention venant d'être exposés ci-dessus dans leur forme la plus élémentaire, d'autres détails et caractéristiques ressortiront plus clairement à la lecture de la description qui suit en regard du dessin annexé illustrant, à titre d'exemple non limitatif, le procédé de détermination de dosage optimal d'un antivitamine K d'un exemple de traitement anticoagulant conforme à l'invention.

La figure portée par ce dessin est une représentation graphique d'un repère cartésien orthonormé avec en axe d'abscisses les unités de temps exprimées en jours et en axe d'ordonnées les unités d'INR.

Le patient ici traité est prescrit en un type d'antivitamine K pour atteindre un INR cible optimal Io fixé par le médecin à 2,8 avec une prescription de départ de surdosage Dmaxi de 28/4 de comprimés par semaine pour une première période T1 puis de sous-dosage Dmini de 21/4 de comprimés par semaine pour une deuxième période T2.

Bien que le sous-dosage Dmini dudit antivitamine K est ici fixé par le prescripteur en même temps que le surdosage Dmaxi, un mode de réalisation préférentiel de l'invention dispose de pouvoir fixer la valeur du sous-dosage Dmini dudit antivitamine K en fonction de la valeur d'INR mesurée au bout de la première période donnée $T_1$ de l'échantillon sanguin administré du surdosage $D_{maxi}$ dudit antivitamine K. En effet, le demandeur a remarqué que plus la valeur de l'INR mesurée au bout de la première période $T_1$ est importante, plus la valeur test du sous-dosage Dmini de l'antivitamines K devra être abaissée.

Sur ce repère graphique, le point A constitue le point de départ du traitement avec un antivitamine K donc avec un temps égal à 0 et un INR de base d'un patient non traité considéré égal à 1.

La valeur $I_o$ de l'INR optimal cible établi à 2,80 par le médecin prescripteur est ensuite représentée en traçant une ligne horizontale en pointillés selon cette valeur 2,80 d'INR.

Au bout d'une première période $T_1$ de quatre jours après le commencement du traitement de trois jours du surdosage $D_{maxi}$ dudit antivitamine K fixé à 28/4 de comprimés par semaine, une valeur de l'INR est mesurée à 2,5 et enregistrée en la reportant sur le repère graphique pour donner le point $I_1$.

La première pente $P_1$ est alors déterminée en traçant une droite qui relie le point A au point $I_1$, pente ascendante correspondant au surdosage $D_{maxi}$ du patient traité à 28/4 de comprimés de l'antivitamine K par semaine.

Cette droite $P_1$ est ensuite prolongée sur une période $T_0$ fixée à 7 jours (une semaine) à partir du 5éme jour (jour où la valeur $I_o$ de l'INR cible de 2,8 est supposée atteinte), permettant d'extrapoler une valeur $I_{maxi}$ de 5,40 de l'INR qui serait obtenue si le surdosage $D_{maxi}$ du patient traité à 28/4 de comprimés dudit antivitamine K avait été maintenu au-delà de l'INR cible 10.

Au bout d'une deuxième période T2 de quatre jours après le commencement d'un nouveau traitement de trois jours du sous-dosage Dmini dudit antivitamine K fixé à 21/4 de comprimés par semaine, et appliqué le 4ème jour soit un jour avant celui où la valeur $I_o$ de l'INR cible fixée à 2,8 serait atteinte sur la pente $P_1$, une deuxième valeur de l'INR est mesurée à 2,5 et enregistrée en la reportant en ordonnée sur le repère graphique pour donner le point $I_2$ déterminée par l'abscisse 8 correspondant au 8ème jour depuis le démarrage du traitement avec l'antivitamine K. Par précaution, la valeur $I_o$ de l'INR cible pourra être vérifiée par une mesure de l'INR à la date prévue, afin de délimiter exactement le point de départ de la deuxième pente $P_2$.

La deuxième pente $P_2$ est alors déterminée en traçant une droite qui relie le point $I_0$ (intersection de la pente $P_2$ avec la valeur $I_o$ de l'INR cible) au point $I_2$, pente descendante correspondant au sous-dosage $D_{mini}$ du patient traité à 21/4 de comprimés de l'antivitamine K par semaine.

Cette droite $P_2$ est ensuite prolongée sur la même période $T_0$ d'une semaine à partir du 5éme jour (moment où la valeur $I_o$ de l'INR cible est atteinte), permettant d'extrapoler une valeur $I_{mini}$ à 2,10 de l'INR qui serait obtenue si le sous-dosage $D_{mini}$ du patient traité à 21/4 de comprimés de l'antivitamine K avait été maintenu au-delà de l'INR cible $I_0$.

Ainsi, à partir d'au moins deux mesures $I_1$ et $I_2$ d'INR reportées sur la représentation graphique illustrée sur le dessin, on peut, avec le procédé de l'invention, et au bout de 8 jours de traitement:

- déterminer une première pente $P_1$ de surdosage $D_{maxi}$ à 28/4 de comprimés et extrapoler un $I_{max}$ de 5,40 de l'INR qui serait obtenu au bout d'une semaine,
- déterminer une deuxième pente $P_2$ de sous-dosage $D_{mini}$ à 21/4 de comprimés et extrapoler un $I_{mini}$ de 2,10 de l'INR qui serait aussi obtenu au bout d'une semaine,
- afin de calculer le facteur F de correction de posologie dudit antivitamine K et la posologie adéquate $D_0$ permettant d'atteindre la valeur $I_o$ de l'INR cible de 2,8 selon les deux formules suivantes de l'invention :

$$F= \frac{I_{maxi} - I_{mini}}{D_{maxi} - D_{mini}} = \frac{5,40 - 2,10}{28/4 - 21/4} = 1,88$$

et

$$D_0 = D_{mini} + \frac{I_0 - I_{mini}}{F} = 21/4 + \frac{2,8 - 2,10}{1,88}$$

$D_0$ = 21/4 +0,37 = 21/4 + 1,49/4 = 22,5/4.

Ainsi, en utilisant un dosage de 22,5/4 par semaine de comprimés de l'antivitamine K, par exemple une semaine à 22/4 et une semaine à 23/4, la courbe d'INR sera alors sensiblement horizontale et très proche voire identique de l'INR cible optimal fixé à 2,80.

Bien évidement, cette détermination du dosage optimal a pour objet essentiel de remplacer les cycles de contrôle et les tâtonnements du médecin prescripteur mais devra être étroitement surveillé en fonction de facteurs extérieurs (alimentation, prise de médicaments, etc....) au traitement de l'antivitamine K qui peuvent influer sur l'INR.

On comprend que le procédé de détermination, qui vient d'être ci-dessus décrit et représenté, l'a été en vue d'une divulgation plutôt que d'une limitation. Bien entendu, divers aménagements, modifications et améliorations pourront être apportés à l'exemple ci-dessus, sans pour autant sortir du cadre de l'invention tel que définie dans les revendications.

Ainsi par exemple, toutes les données à savoir :

- l'INR cible $I_o$ dudit INR optimal cible,
- les valeurs de surdosage $D_{maxi}$ et de sous-dosage $D_{mini}$ de l'antivitamine K,
- les périodes $T_1$ et $T_2$ des mesures des valeurs $I_{maxi}$ et $I_{mini}$ d'INR,
- les valeurs ($I_1$ et $I_2$) d'INR mesurées au cours des périodes $T_1$ et $T_2$,

pourront être saisies directement dans un ordinateur qui, doté d'un programme informatique, sera alors capable, en fonction de ces données $I_o$, $D_{maxi}$, $D_{mini}$, $T_1$, $T_2$, et des mesures des valeurs ($I_1$ et $I_2$) d'INR relevées, de calculer et de déterminer :

- la pente $P_1$ des valeurs d'INR obtenues à partir du surdosage $D_{maxi}$ dudit antivitamine K et la valeur $I_{maxi}$ d'INR théoriquement atteinte à partir du jour où la valeur $I_o$ de l'INR cible est atteinte et au bout d'une période $T_0$,
- la pente $P_2$ des valeurs d'INR obtenues à partir d'un sous-dosage $D_{mini}$ dudit antivitamine K et la valeur $I_{mini}$ d'INR théoriquement atteinte à partir du jour où la valeur $I_o$ de l'INR cible est atteinte et au bout de la période $T_0$,
- le facteur F de correction de posologie d'antivitamines K à partir de la formule :

$$F= \frac{I_{maxi} - I_{mini}}{D_{maxi} - D_{mini}}$$

- et enfin, la posologie adéquate $D_0$ permettant d'atteindre la valeur $I_o$ de l'INR cible fixée à partir de la formule :

$$D_0 = D_{mini} + \frac{I_0 - I_{mini}}{F}.$$

## Revendications

1. Composition pour l'utilisation dans le traitement anticoagulant et déterminée à partir d'un dosage optimal D0 d'un antivitamine K permettant d'atteindre et de maintenir constante la valeur d'un INR cible Io fixé préalablement par le prescripteur en fonction de la pathologie du patient traité, **CARACTERISEE EN CE QUE** le dosage optimal D0 dudit antivitamine K est obtenu à partir d'un procédé qui consiste à :

   - (a) à enregistrer la valeur $I_0$ de l'INR optimal cible,
   - (b) à enregistrer les valeurs d'INR mesurées dans une période donnée $T_1$ à partir d'un surdosage $D_{maxi}$ dudit antivitamine K fixé préalablement par le prescripteur au-dessus du dosage préconisé, afin de déterminer une première pente $P_1$ des valeurs d'INR en fonction du temps $T_1$,
   - (c) à partir de la pente P1, à extrapoler et à enregistrer une valeur $I_{maxi}$ d'INR théoriquement atteinte à partir du jour où la valeur $I_0$ de l'INR cible est atteinte et au bout d'une période déterminée $T_0$,
   - (d) à enregistrer les valeurs d'INR mesurées dans une période donnée $T_2$ à partir d'un sous-dosage $D_{mini}$ dudit antivitamine K fixé par le prescripteur au-dessous du dosage préconisé, afin de déterminer une deuxième pente $P_2$ des valeurs d'INR en fonction du temps $T_2$,
   - (e) à partir de la pente P2, à extrapoler et à enregistrer une valeur $I_{mini}$ d'TNR théoriquement atteinte à partir du jour où la valeur $I_0$ de l'INR cible est atteinte et au bout de la même période déterminée $T_0$,
   - (f) à calculer à partir de la différence entre les valeurs $I_{maxi}$ et $I_{mini}$ d'INR théoriquement calculées, un facteur de correction de posologie dudit antivitamine K exprimé selon la formule suivante :

$$F = \frac{I_{maxi} - I_{mini}}{D_{maxi} - D_{mini}}$$

   - (g) afin de déterminer à partir de la différence entre les valeurs $I_0$ de l'INR cible optimal et $I_{mini}$ de l'INR minimum calculé, et du facteur F de correction de posologie dudit antivitamine K, la posologie adéquate D0 permettant d'atteindre et de maintenir constante la valeur Io de l'INR cible fixée préalablement selon la relation suivante :

$$D_0 = D_{mini} + \frac{I_0 - I_{mini}}{F}.$$

2. Composition selon la revendication 1, **CARACTERISEE EN CE QUE** le sous-dosage Dmini dudit antivitamine K est fixé en fonction de la valeur ($I_1$) d'INR mesurée au bout de la première période donnée $T_1$ de l'échantillon sanguin administré du surdosage $D_{maxi}$ d'antivitamines K.

3. Composition selon la revendication 1, **CARACTERISEE EN CE QUE** le sous-dosage $D_{mini}$ dudit antivitamine K est fixé par le prescripteur, à partir d'un INR cible Io et selon l'état de santé du patient

4. Composition selon la revendication 1, **CARACTERISEE EN CE QUE** l'étape (b) consiste à enregistrer la valeur (I1) d'INR mesurée au bout d'une période T1 de quatre jours après un traitement de trois jours du surdosage Dmaxi dudit antivitamine K, et de déterminer la première pente $P_1$ en considérant qu'à l'instant 0 (point A), la valeur d'INR est égale à 1 correspondant à celle d'un patient non traité en anticoagulant.

5. Composition selon la revendication 1, **CARACTERISEE EN CE QUE** l'étape (d) consiste à enregistrer la valeur ($I_2$)

d'INR mesurée au bout d'une période $T_2$ de quatre jours après un nouveau traitement de trois jours du sous-dosage $D_{mini}$ dudit antivitamine K fixé appliqué le jour avant où la valeur $I_O$ de l'INR cible sera théoriquement atteinte, et de déterminer la deuxième pente $P_2$ en considérant le point de départ à l'intersection de la pente $P_2$ avec la valeur $I_O$ de l'INR cible.

**6.** Composition selon la revendication 1, **CARACTERISEE EN CE QUE** la période déterminée $T_0$ pour extrapoler les deux valeurs $I_{maxi}$ et $I_{mini}$ d'INR théoriquement atteintes est égale à une semaine.

**7.** Composition selon les revendications 1 et 5, **CARACTERISEE EN CE QUE** la valeur $I_O$ de l'INR cible est vérifiée par une mesure de l'INR le jour où théoriquement la valeur $I_O$ de l'INR cible est atteinte selon la pente $P_1$, afin de délimiter exactement le point de départ de la deuxième pente $P_2$.

**8.** Composition selon l'une quelconque des revendications 1 à 7, **CARACTERISEE EN CE QUE** toutes les données à savoir :

    - la valeur $I_O$ dudit INR optimal cible,
    - les valeurs de surdosage Dmaxi et de sous-dosage Dmini dudit antivitamine K,
    - les périodes T1 et T2 des mesures des valeurs Imaxi et Imini d'INR,
    - les valeurs ($I_1$ et $I_2$) d'INR mesurées au cours des périodes $T_1$ et $T_1$,
sont saisies dans un ordinateur doté d'un programme informatique capable, en fonction de ces données $I_O$, $D_{maxi}$, $D_{mini}$, $T_1$ et $T_2$, et des mesures des valeurs $I_1$ et $I_2$ d'INR relevées, de calculer et de déterminer :
    - la pente P1 des valeurs d'INR obtenues à partir du surdosage Dmaxi dudit antivitamine K et la valeur $I_{maxi}$ d'INR théoriquement atteinte à partir du jour où la valeur $I_O$ de l'INR cible est atteinte et au bout d'une période $T_0$,
    - la pente $P_2$ des valeurs d'INR obtenues à partir d'un sous-dosage $D_{mini}$ dudit antivitamine K et la valeur $I_{mini}$ d'INR théoriquement atteinte à partir du jour où la valeur $I_O$ de l'INR cible est atteinte et au bout de la période $T_0$,
    - le facteur F de correction de posologie dudit antivitamine K à partir de la formule :

$$F = \frac{I_{maxi} - I_{mini}}{D_{maxi} - D_{mini}}$$

    - et enfin, la posologie adéquate D0 permettant d'atteindre et de maintenir constante la valeur $I_O$ de l'INR cible fixée à partir de la formule :

$$D_0 = D_{mini} + \frac{I_0 - I_{mini}}{F}.$$

**9.** Composition selon l'une quelconque des revendications 1 à 7, **CARACTERISEE EN CE QUE** toutes les données à savoir :

    - l'INR cible $I_O$ dudit INR optimal cible,
    - les valeurs de surdosage $D_{maxi}$ et de sous-dosage $D_{mini}$ dudit antivitamine K,
    - les périodes $T_1$ et $T_2$ des mesures des valeurs $I_{maxi}$ et $I_{mini}$ d'INR,
    - les valeurs ($I_1$ et $I_2$) d'INR mesurées au cours des périodes $T_1$ et $T_1$,
sont reportées sur une représentation graphique d'un repère cartésien orthonormé avec en axe d'abscisses les unités de
unités de temps et en axe d'ordonnées les unités d'INR permettant de :
    - tracer la pente $P_1$ des valeurs ($I_1$) d'INR obtenues à partir du surdosage $D_{maxi}$ dudit antivitamine K,
    - tracer la pente $P_2$ des valeurs ($I_2$) d'INR obtenues à partir d'un sous-dosage $D_{mini}$ dudit antivitamine K,
    - de visualiser en prolongeant la pente $P_1$, la valeur $I_{maxi}$ d'INR théoriquement atteinte à partir du jour où la valeur $I_O$ de l'INR cible est atteinte et au bout d'une période $T_0$,

- de visualiser en prolongeant la pente $P_2$, la valeur $I_{mini}$ d'INR théoriquement atteinte à partir du jour où la valeur $I_O$ de l'INR cible est atteinte et au bout de la période $T_0$,
- de mesurer sur la période $T_0$, la différence $I_{maxi}$ - $I_{mini}$ et la différence $I_0$- Imini,

afin de calculer selon les deux formules suivantes, le facteur F de correction de posologie dudit antivitamine K et la posologie adéquate $D_0$ permettant d'atteindre et de maintenir constante la valeur Io fixée préalablement:

$$F = \frac{I_{maxi} - I_{mini}}{D_{maxi} - D_{mini}}$$

$$D_0 = D_{mini} + \frac{I_0 - I_{mini}}{F}.$$

## Patentansprüche

1. Zusammensetzung zur Verwendung bei der Antikoagulansbehandlung, von der ausgehend eine optimale Dosierung D0 eines Antivitamins K bestimmt wird, die ermöglicht, den Wert eines Ziel-INR $I_o$, der zuvor von dem verschreibenden Arzt in Abhängigkeit von der Pathologie des behandelten Patienten festgelegt wurde, zu erreichen und konstant zu halten, **dadurch gekennzeichnet, dass** die optimale Dosierung D0 des besagten Antivitamins K ausgehend von einem Verfahren erhalten wird, das in Folgendem besteht:

   - (a) Aufzeichnen des Werts $I_o$ des optimalen Ziel-INR,
   - (b) Aufzeichnen der INR-Werte, die innerhalb eines gegebenen Zeitraums $T_1$ ausgehend von einer Überdosierung $D_{maxi}$ des besagten Antivitamins K, die zuvor von dem verschreibenden Arzt festgelegt wurde, oberhalb der empfohlenen Dosierung gemessen werden, um ein erstes Gefälle $P_1$ von INR-Werten in Abhängigkeit von der Zeit $T_1$ zu bestimmen,
   - (c) ausgehend von dem Gefälle $P_1$ Extrapolieren und Aufzeichnen eines Werts $I_{maxi}$ eines theoretisch erreichten INR ausgehend von dem Tag, an dem der Wert $I_o$ des Ziel-INR erreicht wird, und zum Ende eines bestimmten Zeitraums $T_0$,
   - (d) Aufzeichnen der INR-Werte, die innerhalb eines gegebenen Zeitraums $T_2$ ausgehend von einer Unterdosierung $D_{mini}$ des besagten Antivitamins K, die von dem verschreibenden Arzt festgelegt wurde, unterhalb der empfohlenen Dosierung gemessen werden, um ein zweites Gefälle $P_2$ von INR-Werten in Abhängigkeit von der Zeit $T_2$ zu bestimmen,
   - (e) ausgehend von dem Gefälle $P_2$ Extrapolieren und Aufzeichnen eines Werts $I_{mini}$ eines theoretisch erreichten INR ausgehend von dem Tag, an dem der Wert $I_o$ des Ziel-INR erreicht wird, und zum Ende desselben bestimmten Zeitraums $T_0$,
   - (f) ausgehend von der Differenz zwischen den berechneten Werten $I_{maxi}$ und $I_{mini}$ eines theoretischen INR Berechnen eines Faktors zur Korrektur der Posologie des besagten Antivitamins K, die gemäß der folgenden Formel ausgedrückt wird:

$$F = \frac{I_{max\,i} - I_{min\,i}}{D_{max\,i} - D_{min\,i}},$$

   - (g) um ausgehend von der Differenz zwischen den Werten $I_0$ des optimalen Ziel-INR und $I_{mini}$ des minimalen berechneten INR und dem Faktor F zur Korrektur der Posologie des besagten Antivitamins K die adäquate Posologie D0, die ermöglicht, den Wert $I_o$ des festgelegten Ziel-INR zu erreichen und konstant zu halten, gemäß der folgenden Beziehung zu bestimmen:

$$D_0 = D_{\min i} + \frac{I_0 - I_{\min i}}{F} \cdot$$

**2.** Zusammensetzung nach Anspruch 1, **dadurch gekennzeichnet, dass** die Unterdosierung $D_{\min i}$ des besagten Antivitamins K in Abhängigkeit von dem INR-Wert ($I_1$) festgelegt wird, der zum Ende des ersten gegebenen Zeitraums $T_1$ von der Blutprobe gemessen wird, der die Überdosierung $D_{\max i}$ von Antivitaminen K verabreicht wurde.

**3.** Zusammensetzung nach Anspruch 1, **dadurch gekennzeichnet, dass** die Unterdosierung $D_{\min i}$ des besagten Antivitamins K von dem verschreibenden Arzt ausgehend von einem Ziel-INR $I_0$ und gemäß dem Gesundheitszustand des Patienten festgelegt wird.

**4.** Zusammensetzung nach Anspruch 1, **dadurch gekennzeichnet, dass** der Schritt (b) darin besteht, den INR-Wert (I1) aufzuzeichnen, der zum Ende eines Zeitraums T1 von vier Tagen nach einer Behandlung über drei Tage mit der Überdosierung Dmaxi des besagten Antivitamins K gemessen wird, und das erste Gefälle $P_1$ unter Berücksichtigung des Zeitpunkts 0 (Punkt A) zu bestimmen, wobei der INR-Wert gleich 1 ist, was dem eines Patienten entspricht, der nicht mit Antikoagulans behandelt wurde.

**5.** Zusammensetzung nach Anspruch 1, **dadurch gekennzeichnet, dass** der Schritt (d) darin besteht, den INR-Wert ($I_2$) aufzuzeichnen, der zum Ende eines Zeitraums $T_2$ von vier Tagen nach einer erneuten Behandlung über drei Tage mit der festgelegten Unterdosierung $D_{\min i}$ des besagten Antivitamins K, die an dem Tag angewendet wird, bevor der Wert $I_0$ des Ziel-INR theoretisch erzielt wird, gemessen wird, und das zweite Gefälle $P_2$ unter Berücksichtigung des Ausgangspunkts an der Überschneidung des Gefälles $P_2$ mit dem Wert $I_0$ des Ziel-INR zu bestimmen.

**6.** Zusammensetzung nach Anspruch 1, **dadurch gekennzeichnet, dass** der bestimmte Zeitraum $T_0$ zum Extrapolieren der beiden Werte $I_{\max i}$ und $I_{\min i}$ von theoretisch erreichten INR einer Woche entspricht.

**7.** Zusammensetzung nach den Ansprüchen 1 und 5, **dadurch gekennzeichnet, dass** der Wert $I_0$ des Ziel-INR durch eine Messung des INR an dem Tag verifiziert wird, an dem theoretisch der Wert $I_0$ des Ziel-INR gemäß dem Gefälle $P_1$ erreicht wird, um den Ausgangspunkt des zweiten Gefälles $P_2$ genau abzugrenzen.

**8.** Zusammensetzung nach einem der Ansprüche 1 bis 7, **dadurch gekennzeichnet, dass** alle Daten, nämlich:

- der Wert $I_0$ des besagten optimalen Ziel-INR,
- die Werte der Überdosierung Dmaxi und der Unterdosierung Dmini des besagten Antivitamins K,
- die Zeiträume T1 und T2 von Messungen der INR-Werte Imaxi und Imini,
- die INR-Werte ($I_1$ und $I_2$), die im Verlauf der Zeiträume $T_1$ und $T_1$ gemessen werden,
in einem Computer erfasst werden, der mit einem Computerprogramm ausgestattet ist, der in Abhängigkeit von diesen Daten $I_0$, $D_{\max i}$, $D_{\min i}$, $T_1$ und $T_2$ und Messungen von erhobenen INR-Werten $I_1$ und $I_2$ Folgendes berechnen und bestimmen kann:
- das Gefälle P1 von INR-Werten, die ausgehend von der Überdosierung Dmaxi des besagten Antivitamins K erhalten werden, und den Wert $I_{\max i}$ eines theoretisch erreichten INR ausgehend von dem Tag, an dem der Wert $I_0$ des Ziel-INR erreicht wird, und zum Ende eines Zeitraums $T_0$,
- das Gefälle $P_2$ von INR-Werten, die ausgehend von der Unterdosierung $D_{\min i}$ des besagten Antivitamins K erhalten werden, und den Wert $I_{\min i}$ eines theoretisch erreichten INR ausgehend von dem Tag, an dem der Wert $I_0$ des Ziel-INR erreicht wird, und zum Ende eines Zeitraums $T_0$,
- den Faktor F zur Korrektur der Posologie des besagten Antivitamins K ausgehend von der Formel:

$$F = \frac{I_{\max i} - I_{\min i}}{D_{\max i} - D_{\min i}},$$

- und schließlich die adäquate Posologie D0, die ermöglicht, den Wert $I_0$ des festgelegten Ziel-INR zu erreichen und konstant zu halten, ausgehend von der Formel:

$$D_0 = D_{\min i} + \frac{I_0 - I_{\min i}}{F} \ .$$

**9.** Zusammensetzung nach einem der Ansprüche 1 bis 7, **dadurch gekennzeichnet, dass** alle Daten, nämlich:

- das Ziel-INR $I_o$ des besagten optimalen Ziel-INR,
- die Werte der Überdosierung $D_{maxi}$ und der Unterdosierung $D_{mini}$ des besagten Antivitamins K,
- die Zeiträume $T_1$ und $T_2$ von Messungen der INR-Werte $I_{maxi}$ und $I_{mini}$,
- die INR-Werte ($I_1$ und $I_2$), die im Verlauf der Zeiträume $T_1$ und $T_1$ gemessen werden,

in eine grafische Darstellung eines kartesischen orthonormierten Koordinatensystems eingetragen werden, wobei die Einheiten der Zeiteinheiten auf der x-Achse und die INR-Einheiten auf der y-Achse sind, was Folgendes ermöglicht:

- Verfolgen des Gefälles $P_1$ der INR-Werte ($I_1$), die ausgehend von der Überdosierung $D_{maxi}$ des besagten Antivitamins K erhalten werden,
- Verfolgen des Gefälles $P_2$ der INR-Werte ($I_2$), die ausgehend von der Unterdosierung $D_{mini}$ des besagten Antivitamins K erhalten werden,
- durch Verlängern des Gefälles $P_1$ Visualisieren des Werts $I_{maxi}$ eines theoretisch erreichten INR ausgehend von dem Tag, an dem der Wert $I_o$ des Ziel-INR erreicht wird, und zum Ende eines Zeitraums $T_0$,
- durch Verlängern des Gefälles $P_2$ Visualisieren des Werts $I_{mini}$ eines theoretisch erreichten INR ausgehend von dem Tag, an dem der Wert $I_o$ des Ziel-INR erreicht wird, und zum Ende eines Zeitraums $T_0$,
- Messen der Differenz $I_{maxi}$ - $I_{mini}$ und der Differenz $I_0$ - $I_{mini}$ in dem Zeitraum $T_0$,

um gemäß den zwei folgenden Formeln den Faktor F zur Korrektur der Posologie des besagten Antivitamins K und die adäquate Posologie D0, die ermöglicht, den zuvor festgelegten Wert Io zu erreichen und konstant zu halten, zu berechnen:

$$F = \frac{I_{\max i} - I_{\min i}}{D_{\max i} - D_{\min i}} \ ,$$

$$D_0 = D_{\min i} + \frac{I_0 - I_{\min i}}{F} \ .$$

## Claims

**1.** Composition for use in anticoagulant treatment and determined on the basis of an optimal dosage D0 of an antivitamin K for obtaining and maintaining the value of a target INR Io previously defined by the prescriber according to the treated patient's disease, **characterised in that** the optimal dosage D0 of said antivitamin K is obtained using a method consisting of:

- (a) recording the value $I_o$ of the target optimal INR,
- (b) recording the INR values measured in a given period $T_1$ using an overdosage $D_{max}$ of said antivitamin K previously defined by the prescriber above the recommended dosage, in order to determine a first slope $P_1$ of the INR values as a function of the time $T_1$,
- (c) on the basis of the slope P1, extrapolating and recording an INR value $I_{max}$ theoretically obtained from the day when the value $I_o$ of the target INR is obtained and after a predetermined period To,
- (d) recording the INR values measured in a given period $T_2$ using an underdosage $D_{min}$ of said antivitamin K previously defined by the prescriber below the recommended dosage, in order to determine a second slope $P_2$ of the INR values as a function of the time $T_2$,
- (e) on the basis of the slope P2, extrapolating and recording an INR value $I_{min}$ theoretically obtained from the day when the value $I_o$ of the target INR is obtained and after a predetermined period To,
- (f) calculating on the basis of the difference between the INR values $I_{max}$ and $I_{min}$ theoretically calculated, a dosage correction factor of said antivitamin K expressed according to the following formula:

$$F = \frac{I_{max} - I_{min}}{D_{max} - D_{min}}$$

- (g) in order to determine on the basis of the difference between the values $I_0$ of the optimal target INR and $I_{min}$ of the calculated minimum INR, and the dosage correction factor F of said antivitamin K, the adequate dosage D0 for obtaining and maintaining the value Io of the target INR previously defined according to the following relation:

$$D_0 = D_{min} + \frac{I_0 - I_{min}}{F}$$

2. Composition according to claim 1, **characterised in that** the underdosage $D_{min}$ of said antivitamin K is defined on the basis of the INR value ($I_1$) measured after the first given period $T_1$ of the blood sample administered of the antivitamin K overdosage $D_{max}$.

3. Composition according to claim 1, **characterised in that** the underdosage $D_{min}$ of said antivitamin K is defined by the prescriber, on the basis of a target INR Io and according to the patient's health.

4. Composition according to claim 1, **characterised in that** step (b) consists of recording the INR value (I1) measured after a period T1 of four days after a three-day treatment of the overdosage Dmax of said antivitamin K, and determining the first slope $P_1$, considering that at the time 0 (point A), the INR value is equal to 1 corresponding to that of a patient not under anticoagulant treatment.

5. Composition according to claim 1, **characterised in that** step (d) consists of recording the INR value ($I_2$) measured after a period $T_2$ of four days after a further three-day treatment of the underdosage Dmin of said antivitamin K defined applied on the day before the value $I_0$ of the target INR will theoretically be obtained, and determining the second slope $P_2$ considering the starting point to be at the intersection of the slope $P_2$ with the value $I_0$ of the target INR.

6. Composition according to claim 1, **characterised in that** the predetermined period To for extrapolating the two INR values $I_{max}$ and $I_{min}$ theoretically obtained is equal to one week.

7. Composition according to claims 1 and 5, **characterised in that** the value $I_0$ of the target INR is verified by measuring the INR on the day when theoretically the value $I_0$ of the target INR is obtained according to the slope $P_1$, in order to define precisely the starting point of the second slope $P_2$.

8. Composition according to any of claims 1 to 7, **characterised in that** all the data, i.e.:

   - the value $I_0$ of said target optimal INR,
   - the overdosage Dmax and underdosage Dmin values of said antivitamin K,
   - the periods T1 and T2 of the measurements of the INR values Imax and Imin,
   - the INR values ($I_1$ and $I_2$) measured during the periods $T_1$ and $T_1$,
   are entered in a computer equipped with a computer program capable, on the basis of these data $I_0$, $D_{max}$, $D_{min}$, $T_1$ and $T_2$, and the measurements of the INR values $I_1$ and $I_2$ recorded, of calculating and determining:
   - the slope P1 of the INR values obtained on the basis of the overdosage Dmax of said antivitamin K and the INR value $I_{max}$ theoretically obtained from the day when the value $I_0$ of the target INR is obtained and after a period To,
   - the slope P2 of the INR values obtained using an underdosage $D_{min}$ of said antivitamin K and the INR value $I_{min}$ theoretically obtained from the day when the value $I_0$ of the target INR is obtained and after the period $T_0$,
   - the dosage correction factor F of said antivitamin K based on the formula:

$$F = \frac{I_{max} - I_{min}}{D_{max} - D_{min}}$$

- and finally, the adequate dosage D0 for obtaining and maintaining the value $I_o$ of the target INR defined based on the formula:

$$D_0 = D_{min} + \frac{I_0 - I_{min}}{F}$$

9. Composition according to any of claims 1 to 7, **characterised in that** all the data, i.e.:

- the target INR $I_o$ of said target optimal INR,
- the overdosage $D_{max}$ and underdosage $D_{min}$ values of said antivitamin K,
- the periods $T_1$ and $T_2$ of the measurements of the INR values $I_{max}$ and $I_{min}$,
- the INR values ($I_1$ and $I_2$) measured during the periods $T_1$ and $T_1$,
are recorded on a graphic representation of an orthonormal Cartesian reference with, on the abscissa axis, the units of time and, on the ordinate axis, the INR units for:
- plotting the slope $P_1$ of the INR values ($I_1$) obtained using the overdosage $D_{max}$ of said antivitamin K,
- plotting the slope $P_2$ of the INR values ($I_2$) obtained using an underdosage $D_{min}$ of said antivitamin K,
- viewing by extending the slope $P_1$, the INR value $I_{max}$ theoretically obtained from the day when the value $I_o$ of the target INR is obtained and after a period To,
- viewing by extending the slope $P_2$, the INR value $I_{min}$ theoretically obtained from the day when the value $I_o$ of the target INR is obtained and after the period To,
- measuring over the period To, the difference $I_{max} - I_{min}$ and the difference $I_0 - I_{min}$,
in order to calculate according to the following two formulas, the dosage correction factor F of said antivitamin K and the adequate dosage D0 for obtaining and maintaining the previously defined value Io:

$$F = \frac{I_{max} - I_{min}}{D_{max} - D_{min}}$$

$$D_0 = D_{min} + \frac{I_0 - I_{min}}{F}$$

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- *Recommandations de prescription des AVK,* Janvier 2004, http://www.geocities.com/bpradines/guideAvk.doc
- *Antithrombotic therapy: Ouick reference guide,* Mars 1999, http://www.sign.ac.uk/pdf/qrg36.pdf
- **BRADLOW BA.** Warfarin therapy - a practical guide. *SOUTH AFRICAN MEDICAL JOURNAL,* 27 Août 1983, vol. 64 (9), 317-319
- **WHITE R H ; HONG R ; VENOOK A P ; DASCHBACH M M ; MURRAY W ; MUNGALL D R ; COLEMAN R W.** INITIATION OF WARFARIN THERAPY COMPARISON OF PHYSICIAN DOSING WITH COMPUTER-ASSISTED DOSING. *JOURNAL OF GENERAL INTERNAL MEDICINE,* 1987, vol. 2 (3), 141-148

- **VERGNES C.** Surveillance des traitements antithrombotiques. *REVUE FRANÇAISE DES LABORATOIRES, EDITIONS SCIENTIFIOUES ET MEDICALES ELSEVIER,* 01 Janvier 1995, vol. 1995, ISSN 0338-9898, 89-99
- **HASSO SCHOLZ ; ULRICH SCHWABE.** Taschenbuch der Arzneibehandlung. SPRINGER, 2005
- **CARTER B L ; TAYLOR J W ; BECKER A.** EVALUATION OF THREE DOSAGE-PREDICTION METHODS FOR INITIAL IN-HOSPITAL STABILIZATION OF WARFARIN THERAPY. *CLiNICAL PHARMACY,* 1987, vol. 6 (1), 37-45